(19) Europäisches Patentamt
European Patent Office
Office européen des brevets

(11) Publication number : **0 479 665 A2**

(12) **EUROPEAN PATENT APPLICATION**

(21) Application number : **91402613.3**

(22) Date of filing : **01.10.91**

(51) Int. Cl.$^5$ : **C07D 211/90,** A61K 31/445, A61K 31/44

(30) Priority : **05.10.90 JP 268736/90**
**06.09.91 JP 227413/90**

(43) Date of publication of application :
**08.04.92 Bulletin 92/15**

(84) Designated Contracting States :
**AT BE CH DE DK ES FR GB IT LI LU NL SE**

(71) Applicant : **TEIKOKU CHEMICAL INDUSTRIES CO., LTD.**
**1-18, Kitahorie 1-chome, Nishi-ku**
**Osaka-shi, Osaka (JP)**

(72) Inventor : **Tamura, Toshiaki**
**C-305, 6, Satsukigaoka Higashi**
**Suita-shi, Osaka (JP)**
Inventor : **Fujiwara, Hiromichi**
**18-1, Nakamachi 6-chome, Fujiwaradai, Kita-ku**
**Kobe-shi, Hyogo-ken (JP)**
Inventor : **Tagami, Hiroyuki**
**27-16, Narimasu 4-chome, Itabashi-ku**
**Tokyo (JP)**

(74) Representative : **Orès, Bernard et al**
**Cabinet ORES 6, Avenue de Messine**
**F-75008 Paris (FR)**

(54) **1,4-Dihydropyridine derivatives, method for preparing the same and use thereof.**

(57) A compound represented by the formula (I) :

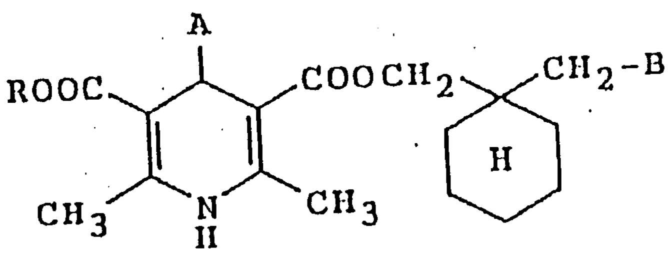

(I)

where A is a phenyl group substituted at least with a nitro group ; B is a group represented by the formula :

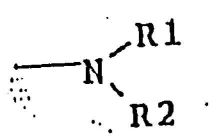

[where $R_1$ and $R_2$ are, the same or different, a lower alkyl group or a benzyl group which may be substituted with a halogen atom, or they may be combined, together with the adjacent nitrogen atom, through or not through an oxygen atom to form a six-membered ring or through a nitrogen atom to form a group represented by the formula :

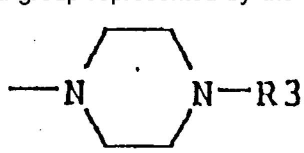

(where $R_3$ is a lower alkyl group, a phenyl or benzyl group which may be substituted with a halogen atom, or a diphenylmethyl group)] ; and R is a hydrogen atom or a lower alkyl group
,or a salt thereof. This compound or a salt thereof is effective as an antihypertensive agent.

EP 0 479 665 A2

BACKGROUND OF THE INVENTION

1. Field of the Invention

This invention relates to novel 1,4-dihydropyridine derivatives, method for preparing the same and use thereof. The compounds of the invention are useful as a cardiovascular agent, particularly an antihypertensive agent.

2. Prior Art

Among 1,4-dihydropyridine derivatives, there have been used nifedipine, nicardipine, nisoldipine, nitrendipine, nilvadipine (general names) and the like for treating patients. These compounds possess a vasodilating action on coronary vessels and cerebral vessels, and are hence useful for treating cerebral blood flow disorders and hypertension. Now, various compounds are subjected to clinical testing, while at the same time attempts to prepare novel compounds and conduct physiological activity tests therefor are repeatedly made.

SUMMARY OF THE INVENTION

The inventors of the present invention have made an intensive research for novel and useful 1,4-dihydropyridine derivatives which are superior to conventional derivatives thereof, and as a result have accomplished the present invention.

Thus, the present invention provides a compound represented by the formula (I):

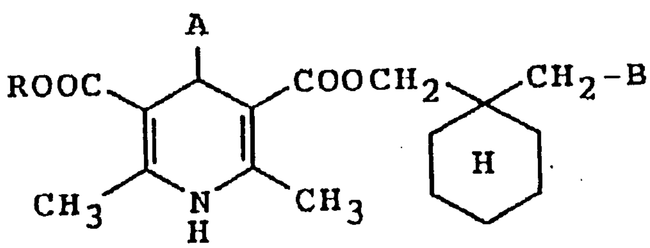

(I)

where A is a phenyl group substituted at least with a nitro group; B is a group represented by the formula:

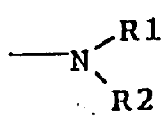

[where $R_1$ and $R_2$ are, the same or different, a lower alkyl group or a benzyl group which may be substituted with a halogen atom, or they may be combined, together with the adjacent nitrogen atom, through or not through an oxygen atom to form a six-membered ring or through a nitrogen atom to form a group represented by the formula:

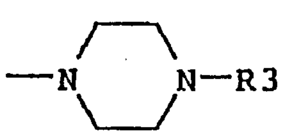

(where $R_3$ is a lower alkyl group, a phenyl or benzyl group which may be substituted with a halogen atom, or a diphenylmethyl group)]; and R is a hydrogen atom or a lower alkyl group
, or a salt thereof.

The present invention also provides a pharmaceutical composition for a circulatory system, which comprises the compound of the above formula (I) or a pharmaceutically acceptable salt thereof, and a pharmaceutically acceptable excipient or carrier.

The present invention further provides a method for preparing the compound of the formula (I) or a salt

thereof.

DETAILED DESCRIPTION OF THE PREFERRED EMBODIMENTS

In the formula (I), the symbol A represents a phenyl group substituted at least with a nitro group. Two or more nitro groups may be present in the phenyl group, but the presence of one nitro group is preferable. In the case of the presence of one nitro group, the phenyl group is preferably substituted at the meta position. The phenyl group may also be substituted with one halogen atom such as fluorine, chlorine or the like.

In the definitions of $R_1$ , $R_2$ and $R_3$ , the lower alkyl group is meant by a straight or branched chain alkyl group having 1-6 carbon atoms and is preferably methyl or ethyl group.

The benzyl group which may be substituted with a halogen atom in $R_1$ , $R_2$ and $R_3$ includes a benzyl group or a benzyl group substituted with one halogen atom such as fluorine, chlorine or the like. The substituted position thereof is preferably the ortho or para position.

The phenyl group which may be substituted with a halogen atom in $R_3$ includes a phenyl group or a phenyl group substituted with one halogen atom such as fluorine, chlorine or the like.

$R_1$ and $R_2$ may be combined, together with the adjacent nitrogen atom, through or not through an oxygen atom to form a six-membered ring, for example, a piperidine ring or morpholine ring. Otherwise, $R_1$ and $R_2$ may be combined, together with the adjacent nitrogen atom, through another nitrogen atom to form a group represented by the following formula:

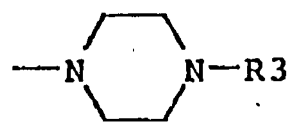

Examples of the group of the above formula include N-methylpiperazinyl, N-ethylpiperazinyl, N-isopropylpiperazinyl, N-phenylpiperazinyl, N-(chlorophenyl)piperazinyl, N-(fluorophenyl)piperazinyl, N-benzylpiperazinyl, N-(chlorobenzyl)piperazinyl, N-(fluorobenzyl)piperazinyl and the like.

The compound of the formula (I) may form its salt with an acid which is preferably a pharmaceutically acceptable salt. Examples of the pharmaceutically acceptable salts of the compound (I) include an inorganic acid salt thereof with a mineral acid such as hydrochloric acid, hydriodic acid, hydrobromic acid, sulfuric acid or the like, and an organic acid salt thereof with an organic sulfonic acid such as methanesulfonic acid, ethanesulfonic acid, toluenesulfonic acid, benzenesulfonic acid or the like, or with an organic carboxylic acid such as lactic acid, glycolic acid, citric acid, succinic acid, tartaric acid, maleic acid, fumaric acid or the like. When R is a hydrogen atom, the compound of the formula (I) may form a salt with, for example, an alkali metal such as sodium or potassium.

The compound of the formula (I) may also form a solvate with a solvent for reaction or recrystallization, upon circumstances. Such a solvate is also included in this invention.

The compound of the formula (I) includes optically active forms thereof based on the asymmetric carbon atom present at 4-position of the dihydropyridine ring. Thus, the compound of the invention includes both racemic forms thereof and optically active forms thereof. Among the optically active forms of the compound of the invention, (+)-forms thereof have turned out to be preferable.

The compound of the formula (I) or a salt thereof can be prepared in accordance with the following methods.

Method (a)

The compound of the formula (I) or a salt thereof can be prepared by reacting a compound of the formula (II):

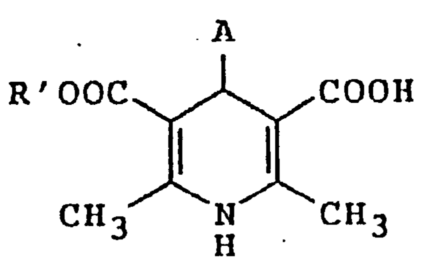

(II)

where A is the same meaning as in the formula (I); R′ represents a lower alkyl group or a protecting group for a carboxyl group
, or a reactive derivative thereof
with a compound of the formula (III):

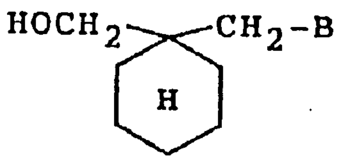

(III)

where B is the same meaning as in the formula (I)
,or a reactive derivative thereof and removing then the protecting group for a carboxyl group, if necessary.

In implementing the above Method (a), the compound of the formula (II) or a reactive derivative thereof is reacted with a chemical equivalent amount of or in slight excess of the compound of the formula (III) or a reactive derivative thereof in an anhydrous organic solvent which is inert against the reaction, at room temperature or under heating.

The reactive derivative of the compound of the formula (II) is meant here by a reactive derivative for carboxyl group which can be ester-linked with the alcoholic OH. Examples of such a reactive derivative include active esters such as p-tolyl ester, p-nitrophenyl ester, phenyl ester, p-methoxyphenyl ester and the like and the acid halides such as chloride, bromide and the like.

When the active ester of the compound (II) is to be used, it is desirable that the reaction be performed under the presence of a base such as an alkali metal hydride (for example, sodium hydride, potassium hydride, lithium hydride and the like), an alkali metal amide (for example, sodium amide and the like), an alkali metal carbonate (for example, potassium carbonate and the like) or the like. Alternatively, when the acid halide of the compound (II) is to be used, it is desirable that the reaction be performed in the presence of a deacidification agent such as an aliphatic or aromatic tertiary amine (for example, triethylamine, dimethylaniline, picoline, pyridine and the like) or an alkali metal carbonate (for example, sodium carbonate, potassium carbonate and the like).

The reactive derivative of the compound of the formula (III) is meant here by a reactive derivative for hydroxyl group which can be ester-linked with the carboxyl group. Examples of such derivatives of the compound of the formula (III) include the halides, sulfonic acid esters (for example, p-toluenesulfonic acid ester, methanesulfonic acid ester and the like) and the like.

Generally, the reaction of the reactive derivative of the compound (II) with the compound of the formula (III) is preferred. In this reaction used as a reaction solvent is tetrahydrofuran, dioxane, toluene, benzene, pyridine or the like.

Otherwise, the reaction of the compound of the formula (II) with the compound of the formula (III) is carried out preferably in the presence of a condensation agent for esterification (for example, N,N′-dicyclohexylcarbodiimide, 1,1′-sulfinyldiimidazol, 1,1′-carbodiimidazol and the like). In this reaction used as a reaction solvent is tetrahydrofuran, dioxane, toluene, benzene, pyridine, dimethylformamide or the like.

The reaction time varies depending on the reaction temperature, the kind of reactant and the like, but is usually a few hours to a few days.

In the case where R′ in the compound of the formula (II) is a protecting group for carboxyl group (for example, trimethylsilyl group, triethylsilyl group and the like), a treatment with an acid or alkali after the reaction enables to easily afford the compound of the formula (I) in which R is a hydrogen atom.

Method (b) and Method (c)

The compound of the formula (I) or a salt thereof can be prepared by Method (b) in which simultaneously reacted are the following three compounds:
a compound of the formula (IV):

A-CHO (IV)

where A is the same meaning as in the formula (I)
,a compound of the formula (V):

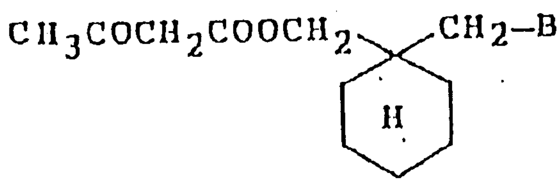

(V)

where B is the same meaning as in the formula (I) ,and β-aminocrotonic acid alkyl ester,
or by Method (c) in which the compound of the formula (IV) is first reacted with the compound of the formula (V) to afford a compound of the formula (VI):

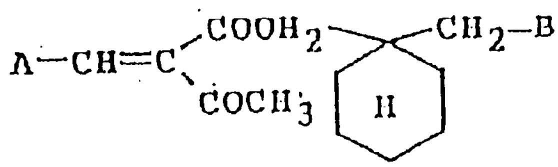

(VI)

where A and B are the same meanings as in the formula (I) ,and then the compound of the formula (VI) is reacted with β-aminocrotonic acid alkyl ester.

Method (b) is effected by reacting the three compounds, namely the compounds of the formulae (IV) and (V) and β-aminocrotonic acid alkyl ester in an organic solvent which is inert against the reaction, under heating. In this reaction it is preferred that the three compounds be substantially chemical equivalent by weight. Examples of the inert organic solvent include isopropylalcohol, tetrahydrofuran, dioxane, benzene, toluene, dimethylformamide, dimethyl sulfoxide, acetonitrile and the like. The reaction time varies depending on the reaction temperature, the kind of reactants and the like, but is usually 1-24 hours.

Method (c) is effected by reacting first the compound of the formula (IV) with the compound of the formula (V) to afford the compound of the formula (VI) and then by reacting β-aminocrotonic acid alkyl ester with the compound of the formula (VI). The reaction solvent used in and the reaction time required for the reaction of the compound of formula (IV) with the compound of the formula (V) are substantially the same as in Method (b). The compound of the formula (VI) as afforded here may be isolated, but is usually reacted with β-aminocrotonic acid alkyl ester under heating without being isolated. In this case ethanol, methanol and the like may be used as a reaction solvent in addition to the aforesaid solvents. The reaction time is usually 1-24 hours.

Method (d)

The compound of the formula (I) or a salt thereof can also be prepared by reacting a compound of the formula (VII):

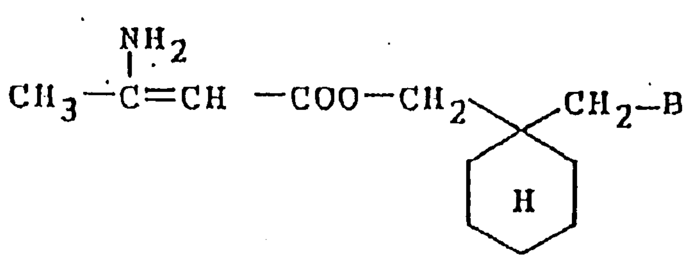

(VII)

with a compound of the formula (VIII):

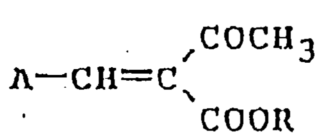

(VIII)

The reaction condition, reaction time and the like for the above reaction are substantially the same as in

Method (c).

Thus, the compound of the formula (I) or a salt thereof is prepared, and can be easily isolated in accordance with a conventional method. The compound of the formula (I) can be converted to a salt thereof by a conventional method, while the salt thereof can be led to another salt thereof by a conventional salt-exchanging method. These compounds may form a solvate with the reaction solvent or recrystallization solvent (for example, isopropyl ether, ethyl ether, acetone, ethyl acetate, chloroform and the like).

The compound of the formula (I) or a salt thereof is preferably a (+) form. The (+) form of the compound (I) or a salt thereof can be obtained by subjecting a (±) form thereof to a conventional optical resolution method. Examples of the resolution agent include cinchonine, D- or L-malic acid, (+)- or (-)-mandelic acid, (+)- or (-)-camphorcarboxylic acid, (+)- or (-)-camphorsulfonic acid, D-(-)-tartaric acid, L-proline, L-lysine, N-acetyl-L-glutamic acid, N-acetyl-L-leucine and the like. Also used in the optical resolution method is such a solvent as methyl alcohol, ethyl alcohol, propyl alcohol, isopropyl alcohol, chloroform, dichloroethane, acetone, dioxane, tetrahydrofuran, water and the like, these solvents being used singly or as an appropriate mixture thereof.

Alternatively, the optically active (+) compound of the formula (I) can be prepared using the optically active (-) compound of the formula (II).

The starting materials used in the invention can be prepared in accordance with a known method or its analogous method. For example, the compound of the formula (II) or an optically active form thereof can be prepared in accordance with the method described in Chem. Pharm. Bull., 28(9), 2809-2812 (1980), or with its analogous method; the compound of the formula (III) can be prepared by the Mannich reaction of cyclohexanecarboxaldehyde with an amine represented by the formula:

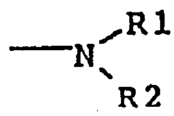

,followed by reduction reaction; the compound of the formula (V) can be prepared by reacting the compound of the formula (III) with diketene; and the compound of the formula (VII) can be prepared by reacting the compound of the formula (V) with ammonia.

The compound of the invention possesses, for example, a potent antihypertensive action and is hence useful as an antihypertensive agent.

Thus, the present invention provides a pharmaceutical composition comprising a compound of the formula (I) or a pharmaceutically acceptable salt thereof and a pharmaceutically acceptable excipient or carrier.

It is preferred that the compound of the invention be orally administered. The dose of the compound through oral administration is usually 0.01-1 g/kg per day, preferably 0.02-0.5 g/kg per day. Such a dose may be given once a day or divided to be given twice or more a day. It would be necessary for individual patients to be administered with an increased or decreased dose depending on the individual's reaction against the active ingredient of the composition or a formulation thereof, the administration time or the administration period. For example, a dose less than the minimum amount of the above dose range may be effective, while on the other hand a dose exceeding the maximum limit of the above dose range may be required. A relatively large amount of dose is preferably divided for several administrations a day.

The formulation of the compound of the invention for oral administration, for example, a tablet can be prepared in accordance with a conventional method in which used are the active ingredient and a pharmaceutically acceptable excipient or carrier such as a binder (for example, corn-starch, polyvinylpyrrolidone, hydroxypropylmethyl cellulose and the like which are all previously gelatinized), an extending agent (for example, lactose, microcrystalline cellulose, calcium phosphate and the like), a lubricant (for example, magnesium stearate, talc, silica and the like), a disintegrator (for example, potato starch, carboxymethyl starch sodium, and the like), or a moistening agent (for exampLe, sodium lauryl sulfate). The tablet may be coated in accordance with a conventional method. The formulation may be in a powdered form. The formulation of the compound of the invention may also be in a liquid form as liquid preparation, syrup or suspension, or in a dried form which is to be added with water or an appropriate adjuvant before the use thereof. Such a liquid formulation can be prepared in accordance with a conventional method by using a pharmaceutically acceptable adjuvant such as a suspending agent (for example, sorbitol syrup, methyl cellulose, hydrogenated oil, and the like), an emulsifier (for example, lecithin, gum arabic and the like), a non-aqueous excipient (almond oil, oily esters, ethyl alcohol and the like) or a preservative (for example, p-hydroxybenzoic acid methyl or propyl ester, sorbic acid).

In the case where the formulation of the compound of the invention is administered as a buccal preparation, the formulation may be in a form of tablet or lozenge which is prepared by a conventional method.

The compound as used in the invention can be formulated for parenteral administration by syringe or for

injection. The injection may be added with a preservative to form a preparation for single administration (for example, ampul) or multiple administration, a liquid preparation, or an emulsion by an oily or aqueous excipient. The injection may also contain an suspending agent, a stabilizer and/or dispersing agent. On the other hand, the active ingredient may be in a powdered form which can be used if added with an appropriate excipient such as sterile water free from pyrogen.

EXAMPLES

The present invention will be described in detail hereinbelow with reference to the Examples which are merely examples and are not limitative of the invention.

Reference Example 1

N-Benzyl-N-methylamine (54.05 g) was dissolved in ethanol (100 ml). The solution was warmed to 50 °C, to which 37 % aqueous formalin solution (36.20 g) was dropwise added. Thereafter, the resultant solution was stirred for 5 minutes at 50 °C. Cyclohexanecarboxaldehyde (50.0 g) was added to the resultant solution, stirred for 8.5 hours under reflux and concentrated under reduced pressure. The residue was dissolved in methanol (100 ml), to which a solution of sodium boron hydride (8.44 g) in water (25 ml) was added dropwise under ice cooling and stirred overnight. The methanol was distilled off under reduced pressure. To the residue was added toluene and water. The organic layer as collected was then washed with water, dried over anhydrous magnesium sulfate and concentrated under reduced pressure. The residue was distilled under reduced pressure, affording 1-(N-benzyl-N-methylaminomethyl)-1-hydroxymethylcyclohexane (85.33 g).

bp: 168 - 171 °C (3 mmHg)

NMR ($CDCL_3$)δ(TMS ppm):

1.40 (10H, S); 2.20 (3H, S); 2.48 (2H, S);
3.50 (2H, S) ; 3.53 (2H, S); 5.9 (1H, S);
7.24 (5H, S)

Reference Example 2

Piperidine (38.00 g) was dissolved in ethanol (100 ml). The solution was warmed to 50 °C, to which 37 % aqueous formalin solution (36.20 g) was dropwise added. Thereafter, the resultant solution was stirred for 5 minutes at 50 °C. Cyclohexanecarboxaldehyde (50.0 g) was added to the resultant solution, stirred for 9 hours under reflux and concentrated under reduced pressure. The residue was dissolved in methanol (100 ml), to which a solution of sodium boron hydride (8.44 g) in water (25 ml) was added dropwise under ice cooling and stirred overnight. The methanol was distilled off under reduced pressure. To the residue was added toluene and water. The organic layer was then washed with water, dried over anhydrous magnesium sulfate and concentrated under reduced pressure. The residue was distilled under reduced pressure, affording 1-piperidinylmethyl-1-hydroxymethylcyclohexane (62.30 g).

bp: 143 - 150 °C (11 mmHg)

NMR ($CDCL_3$)δ(TMS ppm):

1.1-1.8 (16H); 2.33 (2H, S); 2.3-2.7 (4H);
3.57 (2H, S) ; 6.50 (1H, S)

Reference Example 3

1-Phenylpiperazine (105.9 g) was dissolved in ethanol (210 ml). The solution was warmed to 50 °C, to which 37 % aqueous formalin solution (53.0 g) was dropwise added. Thereafter, the resultant solution was stirred for 5 minutes at 50 °C. Cyclohexanecarboxaldehyde (73.2 g) was added to the resultant solution, stirred for 9 hours under reflux and concentrated under reduced pressure. The residue was dissolved in methanol (525 ml), to which a solution obtained by dissolving sodium boron hydride (9.30 g) in water (30 ml) was added dropwise under ice cooling and stirred for 3 hours. The methanol was distilled off under reduced pressure. To the residue was added toluene and water. The organic layer was then washed with water, dried over anhydrous magnesium sulfate and concentrated under reduced pressure. Hexane was added to the residue, affording 1-(N-phenylpiperazinylmethyl)-1-hydroxymethylcyclohexane (108.5 g) as crystals.

mp: 60 - 61 °C

NMR ($CDCL_3$)δ(TMS ppm):

1.43 (10H, S); 2.47 (2H, S); 2.6-2.9 (4H);

3.0-3.4 (4H) ; 3.62 (2H, S); 5.8 (1H, S);
6.8-7.5 (5H)

Example 1 [Method (a)]

N,N′-Dicyclohexylcarbodiimide (1.02 g) was added to a mixture of 1,4-dihydro-2,6-dimethyl-4-(3′-nitrophenyl)-3,5-pyridinedicarboxylic acid monomethyl ester (1.50 g), 1-(N-benzyl-N-methylaminomethyl)-1-hydroxymethylcyclohexane (1.23 g) and pyridine (4.5 ml). The resultant mixture was stirred for 1.5 hours at 80 °C. After cooling, chloroform was added to the reaction mixture. The reaction mixture was adjusted to pH 1 with diluted hydrochloric acid and stirred for 30 minutes. After filtering the insoluble substances off, the chloroform layer was collected, washed with water, aqueous potassium carbonate solution and water in this order, dried over anhydrous magnesium sulfate and concentrated under reduced pressure. The residue was subjected to a silica gel chromatography (toluene : ethyl acetate = 4 : 1), affording oil (1.95 g).

The obtained oil was dissolved in chloroform, to which hydrochloric acid-isopropyl ether was added to form the hydrochloride. The resultant solution was concentrated under reduced pressure. The residue was crystallized from acetone, affording 1,4-dihydro-2,6-dimethyl-4-(3′-nitrophenyl)-3,5-pyridinedicarboxylic acid 1-(N-benzyl-N-methylaminomethyl)cyclohexylmethyl methyl ester hydrochloride (1.99g).

Example 2 [Method (a)]

N,N′-Dicyclohexylcarbodiimide (1.02 g) was added to a mixture of 1,4-dihydro-2,6-dimethyl-4-(3′-nitrophenyl)-3,5-pyridinedicarboxylic acid monomethyl ester (1.50 g), 1-piperidinylmethyl-1-hydroxymethylcyclohexane (1.05 g) and pyridine (4.5 ml). The resultant mixture was stirred for 1.5 hours at 90 °C. After cooling, chloroform was added to the reaction mixture. The reaction mixture was adjusted to pH 1 with diluted hydrochloric acid and stirred for 30 minutes. After filtering the insoluble substances off, the chloroform layer was washed with water, aqueous potassium carbonate solution and water in this order, dried over anhydrous magnesium sulfate and concentrated under reduced pressure. The residue was subjected to a silica gel chromatography (toluene : ethyl acetate = 4 : 1), affording oil (1.96 g).

The obtained oil was dissolved in chloroform, to which hydrochloric acid-isopropyl ether was added to form the hydrochloride. The resultant solution was concentrated under reduced pressure. The residue was crystallized from acetone, affording 1,4-dihydro-2,6-dimethyl-4-(3′-nitrophenyl)-3,5-pyridinedicarboxylic acid methyl (1-piperidinylmethylcyclohexyl)methyl ester hydrochloride (1.98g).

Example 3 [Method (a)]

N,N′-Dicyclohexylcarbodiimide (1.37 g) was added to a mixture of 1,4-dihydro-2,6-dimethyl-4-(3′-nitrophenyl)-3,5-pyridinedicarboxylic acid monomethyl ester (2.00 g), 1-(4-phenylpiperazinylmethyl)-1-hydroxymethylcyclohexane (1.91 g) and pyridine (6.0 ml). The resultant mixture was stirred for 3 hours at 90 °C. After cooling, chloroform was added to the reaction mixture. The reaction mixture was adjusted to pH 1 with diluted hydrochloric acid and stirred for 30 minutes. The insoluble substances were filtered off. The chloroform layer was washed with water, aqueous potassium carbonate solution and water in this order, dried over anhydrous magnesium sulfate and concentrated under reduced pressure. The residue was subjected to a silica gel chromatography (toluene : ethyl acetate = 4 : 1), affording oil (2.87 g).

The obtained oil was dissolved in chloroform, to which hydrochloric acid-isopropyl ether was added to form the hydrochloride. The resultant solution was concentrated under reduced pressure. The residue was crystallized from acetone, affording 1,4-dihydro-2,6-dimethyl-4-(3′-nitrophenyl)-3,5-pyridinedicarboxylic acid methyl 1-(4-phenylpiperazinylmethyl)cyclohexylmethyl ester dihydrochloride·1/2 acetone adduct (2.12 g).

Examples 4 - 17

Compounds of Examples 4 to 17 were prepared by the same manner as in Example 1.

Example 18

N,N′-Dicyclohexylcarbodiimide (1.02 g) was added to a mixture of (-)-1,4-dihydro-2,6-dimethyl-4-(3′-nitrophenyl)-3,5-pyridinedicarboxylic acid monomethyl ester (1.50 g), 1-(N-benzyl-N-methylaminomethyl)-1-hydroxymethylcyclohexane (1.23 g) and pyridine (4.5 ml). The resultant mixture was stirred for 1.5 hours at 80 °C. After cooling, chloroform was added to the reaction mixture. The reaction mixture was adjusted to pH 1 with

diluted hydrochloric acid and stirred for 30 minutes. The insoluble substances were filtered off. The chloroform layer was washed with water, aqueous potassium carbonate solution and water in this order, dried over anhydrous magnesium sulfate and concentrated under reduced pressure. The residue was subjected to a silica gel chromatography (toluene : ethyl acetate = 4:1), affording oil (2.06 g).

The obtained oil was dissolved in chloroform, to which hydrochloric acid-isopropyl ether was added to form the hydrochloride. The resultant solution was concentrated under reduced pressure. The residue was crystallized from acetone, affording (+)-1,4-dihydro-2,6-dimethyl-4-(3′-nitro-phenyl)-3,5-pyridinedicarboxylic acid 1-(N-benzyl-N-methylaminomethyl)cyclohexylmethyl methyl ester hydrochloride (2.09 g).

Example 19

N,N′-Dicyclohexylcarbodiimide (0.68 g) was added to a mixture of (-)-1,4-dihydro-2,6-dimethyl-4-(3′-nitrophenyl)-3,5-pyridinedicarboxylic acid monomethyl ester (1.00 g), 1-piperidinylmethyl-1-hydroxymethylcyclohexane (0.70 g) and pyridine (3.0 ml). The resultant mixture was stirred for 2 hours at 90 °C. After cooling, chloroform was added to the reaction mixture. The reaction mixture was adjusted to pH 1 with diluted hydrochloric acid and stirred for 30 minutes. The insoluble substances were filtered out. The chloroform layer was washed with water, aqueous potassium carbonate solution and water in this order, dried over anhydrous magnesium sulfate and concentrated under reduced pressure. The residue was subjected to a silica gel chromatography (toluene : ethyl acetate = 4 : 1), affording oil (1.22 g).

The obtained oil was dissolved in chloroform, to which hydrochloric acid-isopropyl ether was added to form the hydrochloride. The resultant solution was concentrated under reduced pressure. The residue was crystallized from acetone, affording (+)-1,4-dihydro-2,6-dimethyl-4-(3′-nitro-phenyl)-3,5-pyridinedicarboxylic acid methyl (1-piperidinylmethylcyclohexyl)methyl ester hydrochloride (1.24 g).

Example 20

N,N′-Dicyclohexylcarbodiimide (1.37 g) was added to a mixture of (-)-1,4-dihydro-2,6-dimethyl-4-(3′-nitrophenyl)-3,5-pyridinedicarboxylic acid monomethyl ester (2.00 g), 1(4-phenylpiperazinylmethyl)-1-hydroxymethylcyclohexane (1.91 g) and pyridine (6.0 ml). The resultant mixture was stirred for 3 hours at 90 °C. After cooling, chloroform was added to the reaction mixture. The reaction mixture was adjusted to pH 1 with diluted hydrochloric acid and stirred for 30 minutes to remove the insoluble substances. The chloroform layer was washed with water, aqueous potassium carbonate solution and water in this order, dried over anhydrous magnesium sulfate and concentrated under reduced pressure. The residue was subjected to a silica gel chromatography (toluene : ethyl acetate = 4 : 1), affording oil (3.04 g).

The obtained oil was dissolved in chloroform, to which hydrochloric acid-isopropyl ether was added to form the hydrochloride. The resultant solution was concentrated under reduced pressure. The residue was crystallized from acetone, affording (+)-1,4-dihydro-2,6-dimethyl-4-(3′-nitro-phenyl)-3,5-pyridinedicarboxylic acid methyl 1-(4-phenylpiperazinylmethyl)cyclohexylmethyl ester dihydrochloride (1.91 g).

Examples 21 - 27

Compounds of Examples 21 to 27 were prepared by the same manner as in Example 18 except for using hydroxymethylcyclohexane derivatives having substituents shown in column B, Table 1.

Examples 28 - 37

Compounds of Examples 28 to 37 were prepared by the same manner as in Example 18 except for employing (+) form of the following compound as a material.

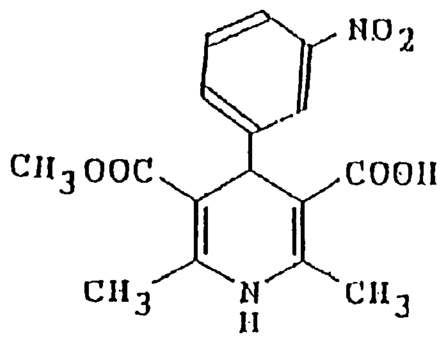

Example 38 [Method (b)]

A mixture of 3-nitrobenzaldehyde (3.02 g), ethyl β-aminocrotonate (2.58 g), acetoacetic acid [1-(N-benzyl-N-methylaminomethyl)cyclohexyl) methyl ester (6.69 g) and isopropylalcohol (15 ml) was stirred for 12 hours under reflux. The reaction solution was concentrated under reduced pressure. The residue was dissolved in chloroform, washed with diluted hydrochloric acid and water successively. The resultant solution was further washed with aqueous sodium hydrogen carbonate solution and water successively, dried over anhydrous magnesium sulfate and concentrated under reduced pressure. The residue was subjected to a silica gel chromatography eluting with toluene-ethyl acetate to afford 1,4-dihydro-2,6-dimethyl-4-(3′-nitrophenyl)-3,5-pyridinedicarboxylic acid 1-(N-benzyl-N-methylaminomethyl)cyclohexylmethyl ethyl ester (2.85 g).

NMR stectrum and IR spectrum of this compound and melting point of the hydrochloride thereof are the same as those obtained in Example 6.

Example 39 [Method (c)]

A mixture of 3-nitrobenzaldehyde (3.02 g), acetoacetic acid [1-(N-benzyl-N-methylaminomethyl)cyclohexyl]methylester (6.96 g), acetic acid (0.06 g), piperidine (0.09 g) and isopropylalcohol (15 ml) was stirred for 24 hours at room temperature. Thereafter, the resultant mixture was concentrated under reduced pressure, affording crude 2-(3′nitrobenzylidene) acetoacetic acid [1-(N-benzyl-N-methylaminomethyl)cyclohexyl]methyl ester. To this compound was added ethyl β-aminocrotonate (2.58 g) and toluene (30 ml). The resultant solution was subjected to dehydration under reflux for 9 hours by using Dean-Stark's apparatus. The reaction solution was washed with aqueous sodium hydrogen carbonate solution and water, dried over anhydrous magnesium sulfate and concentrated under reduced pressure. The residue was subjected to a silica gel chromatography eluting with toluene-ethyl acetate to afford 1,4-dihydro-2,6-dimethyl-4-(3′-nitrophenyl)-3,5-pyridinedicarboxylic acid 1-(N-benzyl-N-methylaminomethyl)cyclohexylmethyl ethyl ester (5.25 g).

NMR stectrum and IR spectrum of this compound and melting point of the hydrochloride thereof are the same as those obtained in Example 6.

Example 40 [Method (d)]

15 % ammonium methanol solution (1.84 g) was dropwise added to acetoacetic acid [1-(N-benzyl-N-methylaminomethyl)cyclohexyl] methyl ester (5.0 g) under ice-cooling. The resultant solution was stirred for an hour and then for 4 hours at room temperature. The resultant solution was concentrated under reduced pressure to obtain crude β-aminocrotonic acid [1-(N-benzyl-N-methylaminomethyl)cyclohexyl] methyl ester. To this compound were added 2-(3′-nitrobenzylidene) acetoacetic acid isopropyl ester (4.18 g) and isopropylalcohol (15 ml) and stirred for 7 hours under reflux. The reaction solution was concentrated under reduced pressure. The residue was dissolved in chloroform and washed with diluted hydrochloric acid and water successively. The resultant solution was further washed with aqueous sodium hydrogen carbonate solution and water, dried over anhydrous magnesium sulfate and concentrated under reduced pressure. The residue was subjected to a silica gel chromatography eluting with toluene-ethyl acetate to afford 1,4-dihydro-2,6-dimethyl-4-(3′-nitrophenyl)-3,5-pyridinedicarboxylic acid 1-(N-benzyl-N-methylaminomethyl)cyclohexyl- methyl isopropyl ester (6.34 g).

NMR stectrum and IR spectrum of this compound and melting point of the hydrochloride thereof are the same as those obtained in Example 4.

The compounds prepared in each Example and property values thereof are shown in Tables 1 and 2.

Table 1

| Ex. No. | A | B | R | Method |
|---|---|---|---|---|
| 1 | 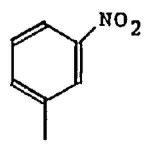 | 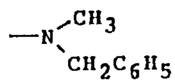 | $CH_3$ | a |
| 2 | 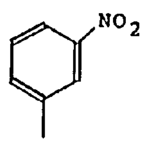 | 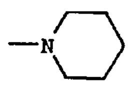 | $CH_3$ | a |
| 3 | 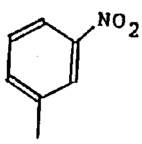 | 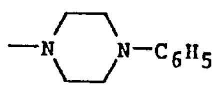 | $CH_3$ | a |
| 4 | 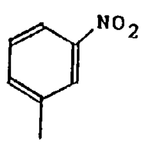 | 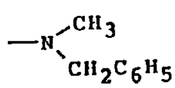 | $CH(CH_3)_2$ | a,d |
| 5 | 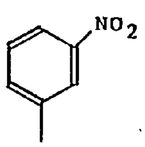 | 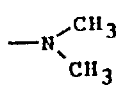 | $CH(CH_3)_2$ | a |
| 6 | 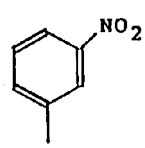 | 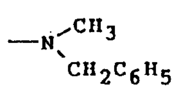 | $CH_2CH_3$ | a,b,c |

Table 1 (continued)

| | | | | |
|---|---|---|---|---|
| 7 | 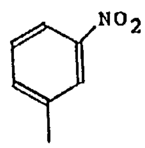 | 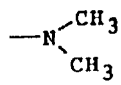 | $CH_2CH_3$ | a |
| 8 | 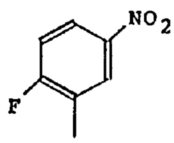 | 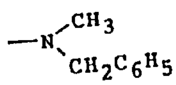 | $CH_3$ | a |
| 9 | 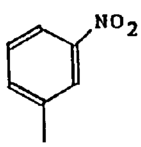 | 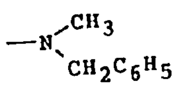 | $CH_3$ | a |
| 10 | 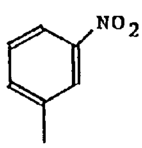 | 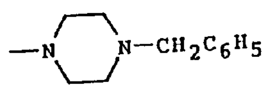 | $CH_3$ | a |
| 11 | 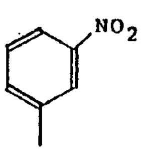 | 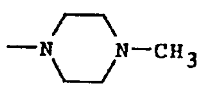 | $CH_3$ | a |
| 12 | 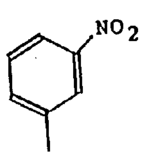 | 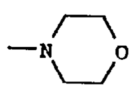 | $CH_3$ | a |
| 13 | 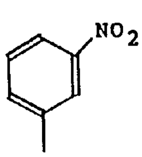 | 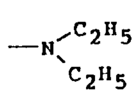 | $CH_3$ | a |
| 14 | 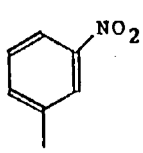 | 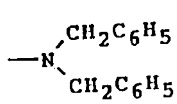 | $CH_3$ | a |

Table 1 (continued)

| | | | | |
|---|---|---|---|---|
| 15 | 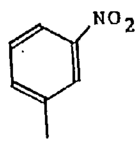 | 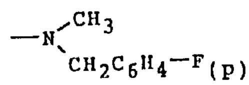 | $CH_3$ | a |
| 16 | 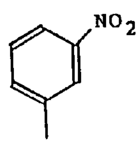 | 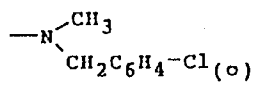 | $CH_3$ | a |
| 17 | 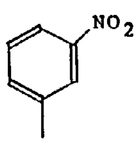 | 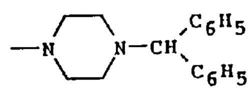 | $CH_3$ | a |
| 18 | 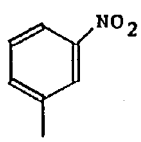 | 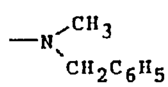 | $CH_3$ | a |
| 19 | 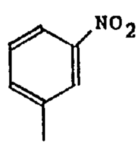 | 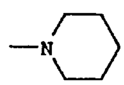 | $CH_3$ | a |
| 20 | 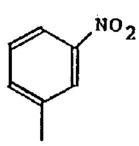 | 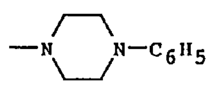 | $CH_3$ | a |
| 21 | 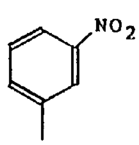 | 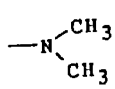 | $CH_3$ | a |
| 22 | 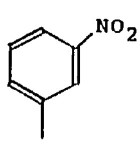 | 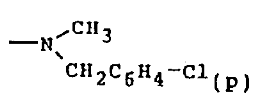 | $CH_3$ | a |

Table 1 (continued)

| | | | | |
|---|---|---|---|---|
| 23 | 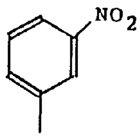 | 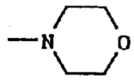 | $CH_3$ | a |
| 24 | 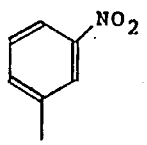 | 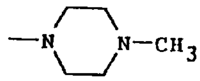 | $CH_3$ | a |
| 25 | 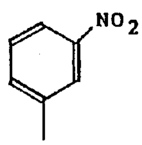 | 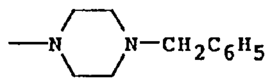 | $CH_3$ | a |
| 26 | 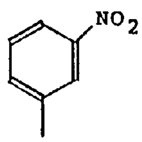 | 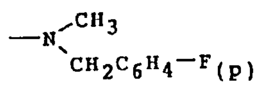 | $CH_3$ | a |
| 27 | 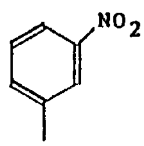 | 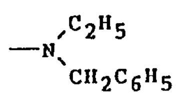 | $CH_3$ | a |
| 28 | 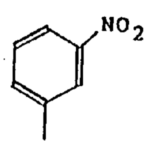 | 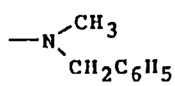 | $CH_3$ | a |
| 29 | 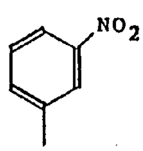 | 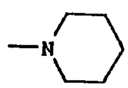 | $CH_3$ | a |
| 30 | 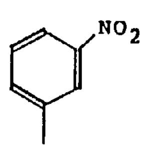 | 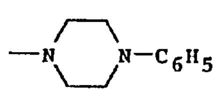 | $CH_3$ | a |

Table 1 (continued)

| | | | | |
|---|---|---|---|---|
| 31 | 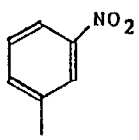 | 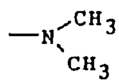 | $CH_3$ | a |
| 32 | 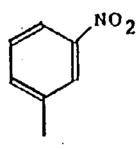 | 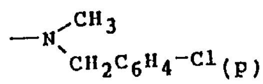 | $CH_3$ | a |
| 33 | 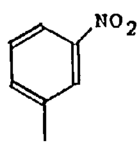 | 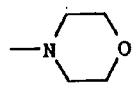 | $CH_3$ | a |
| 34 | 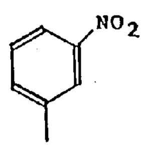 | 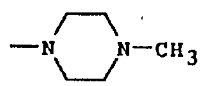 | $CH_3$ | a |
| 35 | 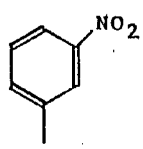 | 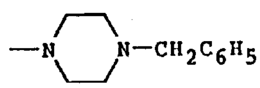 | $CH_3$ | a |
| 36 | 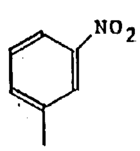 | 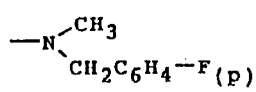 | $CH_3$ | a |
| 37 | 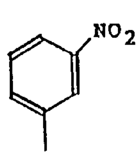 | 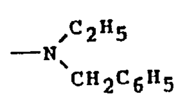 | $CH_3$ | a |
| | | | | |

Table 2

| Ex. No. | NMR Spectrum | Salt | | |
|---|---|---|---|---|
| | | Hydrochloride | mp(C°) | $[\alpha]_D^{20}$ c=1, methanol |
| 1 | 1.35(10H,S) 2.02(3H,S) 2.27,2.37(8H,S,S) 3.38(2H,S) 3.63(3H,S) 4.07(2H,S) 5.13(1H,S) 6.12(1H,S) 7.0 ~ 8.3(9H) | HCl | 137 ~ 138 | ± |
| 2 | 1.33(10H,S) 1.9 ~ 2.6(12H) 3.70(3H,S) 4.00(2H,S) 5.20(1H,S) 6.13(1H,S) 7.2 ~ 8.3(4H) | HCl | 184 ~ 188 | ± |
| 3 | 1.35(10H,S) 2.20(2H,S) 2.28(3H,S) 2.40(3H,S) 2.2 ~ 2.7(4H) 2.8 ~ 3.3(4H) 3.67(3H,S) 4.0(2H,S) 5.17(1H,S) 6.32(1H,S) 6.6 ~ 8.3(9H) | 2HCl 1/2 acetone | 161 ~ 164 | ± |
| 4 | 1.15(3H,d) 1.25(3H,d) 1.35(10H,S) 2.00(3H,S) 2.17 ~ 2.50(8H) 3.35(2H,S) 4,02(2H,S) 4.95(1H,sep) 5.12(1H,S) 6.27(1H,S) 6.9 ~ 8.2(9H) | HCl 1/2 acetone | 145 ~ 148 | ± |
| 5 | 1.20(3H,d) 1.30(3H,d) 1.35(10H,S) 2.15(8H,S) 2.30(3H,S) 2.42(3H,S) 3.98(2H,S) 5.02(1H,sep) 5.18(1H,S) 6.38(1H,S) 7.1 ~ 8.3(4H) | HCl 1/2 IPE | 99 ~ 103 | ± |
| 6 | 1.27(3H,t) 1.35(10H,S) 2.02(3H,S) 2.20 ~ 2.50(8H) 3.37(2H,S) 4.05(2H,S) 4.10(2H,q) 5.15(1H,S) 6.27(1H,S) 7,0 ~ 8.3(9H) | HCl | 175 ~ 178 | ± |

Table 2 (continued)

| | | | | |
|---|---|---|---|---|
| 7 | 1.28(3H,t) 1.33(10H,S) 2.13(8H,S) 2.30(3H,S) 2.40(3H,S) 3.97(2H,S) 4.13(2H,q) 5.17(1H,S) 6.38(1H,S) 7.1 - 8.3(4H) | HCl 1/3 IPE | 103 - 106 | ± |
| 8 | 1.37(10H,S) 2.03(3H,S) 2.2 - 2.5(8H) 3.40(2H,S) 3.60(3H,S) 4.07(2H,S) 5.33(1H,S) 6.48(1H,S) 6.7 - 7.5(6H) 7.7 - 8.3(2H) | HCl 1/2 IPE | 129 - 132 | ± |
| 9 | 0.83(3H,t) 1.33(10H,S) 2.27(3H,S) 2.35(3H,S) 2.37(2H,q) 3.47(2H,S) 3.63(3H,S) 4.03(2H,S) 5.12(1H,S) 6.22(1H,S) 7.0 - 8.2(9H) | HCl 1/3 acetone | 140 - 142 | ± |
| 10 | 1.33(10H,S) 2.13(2H,S) 2.1 - 2.6 (14H) 3.10(2H,S) 3.67(3H,S) 3.97(2H,S) 5.15(1H,S) 6.07(1H,S) 7.1 - 8.2(9H) | 2HCl | 150 - 153 | ± |
| 11 | 1.33(10H,S) 1.8 - 2.7(19H) 3.68(3H,S) 3.97(2H,S) 5.17(1H,S) 6.17(1H,S) 7.1 - 8.3(4H) | 2HCl 1/3 acetone | 153 - 157 | ± |
| 12 | 1.33(10H,S) 1.9 - 2.8(12H) 3.3 - 3.9(4H) 3.67(3H,S) 3.97(2H,S) 5.13(1H,S) 6.73(1H,S) 7.1 - 8.2(4H) | HCl | 154 - 156 | ± |
| 13 | 0.87(6H,t) 1.37(10H,S) 2.0 - 2.9(12H) 3.67(3H,S) 3.97(2H,S) 5.15(1H,S) 6.40(1H,S) 7.1 - 8.3(4H) | HCl | 152 - 154 | ± |
| 14 | 1.27(10H,S) 2.30(6H,S) 2.48(2H,S) 3.47(4H,S) 3.67(3H,S) 4.00(2H,S) 5.08(1H,S) 6.07(1H,S) 7.22(10H,S) 7.0 - 8.3(4H) | HCl | 138 - 140 | ± |

Table 2 (continued)

| | | | | |
|---|---|---|---|---|
| 15 | 1.37(10H,S) 2.03(3H,S) 2.1 - 2.7(8H) 2.35(2H,S) 3.60(3H,S) 4.07(2H,S) 5.15(1H,S) 6.23(1H,S) 6.7 - 8.3(8H) | HCl | 138 - 142 | ± |
| 16 | 1.37(10,S) 2.10(3H,S) 2.0 - 2.7(8H) 3.52(2H,S) 3.65(3H,S) 4.02(2H,S) 5.13(1H,S) 6.68(1H,S) 6.9 - 8.3(8H) | HCl 1/6 acetone | 180 - 183 | ± |
| 17 | 1.30(10H,S) 1.9 - 2.7(16H) 3.63(3H,S) 3.98(2H,S) 4.13(1H,S) 5.17(1H,S) 6.37(1H,S) 7.0 - 8.3(14H) | 2HCl | 162 - 165 | ± |
| 18 | 1.35(10H,S) 2.02(3H,S) 2.27,2.37(8H,S,S) 3.38(2H,S) 3.63(3H,S) 4.07(2H,S) 5.13(1H,S) 6.12(1H,S) 7.0 - 8.3(9H) | HCl | 136 - 138 | +71.65 |
| 19 | 1.33(10H,S) 1.9 - 2.6(12H) 3.70(3H,S) 4.00(2H,S) 5.20(1H,S) 6.13(1H,S) 7.2 - 8.3(4H) | HCl | 202 - 205 | +78.19 |
| 20 | 1.35(10H,S) 2.20(2H,S) 2.28(3H,S) 2.40(3H,S) 2.2 - 2.7(4H) 2.8 - 3.3(4H) 3.67(3H,S) 4.00(2H,S) 5.17(1H,S) 6.32(1H,S) 6.6 - 8.3(9H) | 2HCl | 176 - 180 | +69.19 |
| 21 | 1.33(10H,S) 2.13(8H,S) 2.30(3H,S) 2.40(3H,S) 3.67(3H,S) 3.98(2H,S) 5.17(1H,S) 6.62(1H,S) 7.1 - 8.3(4H) | HCl | 141 - 143 | +79.49 |
| 22 | 1.33(10H,S) 2.00(3H,S) 2.27,2.37(8H,S,S) 3.32(2H,S) 3.65(3H,S) 4.02(2H,S) 5.10(1H,S) 6.22(1H,S) 7.0 - 8.3(8H) | HCl | 158 - 161 | +61.53 |

Table 2 (continued)

| | | | | |
|---|---|---|---|---|
| 23 | 1.33(10H,S) 1.9 - 2.8(12H) 3.3 - 3.9(4H) 3.67(3H,S) 3.97(2H,S) 5.13(1H,S) 6.73(1H,S) 7.1 - 8.2(4H) | HCl | 160 - 162 | +78.59 |
| 24 | 1.33(10H,S) 1.8 - 2.7(19H) 3.68(3H,S) 3.97(2H,S) 5.17(1H,S) 6.17(1H,S) 7.1 - 8.3(4H) | 2HCl | 136 - 139 | +71.59 |
| 25 | 1.33(10H,S) 2.13(2H,S) 2.1 - 2.6(14H) 3.10(2H,S) 3.67(3H,S) 3.97(2H,S) 5.15(1H,S) 6.07(1H,S) 7.1 - 8.2(9H) | 2HCl 1/10 acetone | 146 - 150 | +59.79 |
| 26 | 1.37(10H,S) 2.03(3H,S) 2.1 - 2.7(8H) 2.35(2H,S) 3.68(3H,S) 4.07(2H,S) 5.15(1H,S) 6.23(1H,S) 6.7 - 8.3(8H) | HCl | 141 - 144 | +73.89 |
| 27 | 0.83(3H,t) 1.33(10H,S) 2.27(3H,S) 2.35(3H,S) 2.37(2H,q) 3.47(2H,S) 3.63(3H,S) 4.03(2H,S) 5.12(1H,S) 6.22(1H,S) 7.0 - 8.2(9H) | HCl | 131 - 134 | +80.59 |
| 28 | 1.35(10H,S) 2.02(3H,S) 2.27, 2.37(8H,S,S) 3.38(2H,S) 3.63(3H,S) 4.07(2H,S) 5.13(1H,S) 6.12(1H,S) 7.0 - 8.3(9H) | HCl | 136 - 138 | -74.89 |
| 29 | 1.33(10H,S) 1.9 - 2.6(12H) 3.70(3H,S) 4.00(2H,S) 5.20(1H,S) 6.13(1H,S) 7.2 - 8.3(4H) | HCl | 202 - 205 | -79.49 |
| 30 | 1.35(10H,S) 2.20(2H,S) 2.28(3H,S) 2.40(3H,S) 2.2 - 2.7(4H) 2.8 - 3.3(4H) 3.67(3H,S) 4.00(2H,S) 5.17(1H,S) 6.32(1H,S) 6.6 - 8.3(9H) | 2HCl | 175 - 181 | -69.29 |

Table 2 (continued)

| | | | | |
|---|---|---|---|---|
| 31 | 1.33(10H,S) 2.13(8H,S) 2.30(3H,S) 2.40(3H,S) 3.67(3H,S) 3.98(2H,S) 5.17(1H,S) 6.62(1H,S) 7.1 - 8.3(4H) | HCl | 140 - 142 | -80.34 |
| 32 | 1.33(10H,S) 2.00(3H,S) 2.27,2.37(8H,S,S) 3.32(2H,S) 3.65(3H,S) 4.02(2H,S) 5.10(1H,S) 6.22(1H,S) 7.0 - 8.3 (8H) | HCl | 157 - 161 | -61.73 |
| 33 | 1.33(10H,S) 1.9 - 2.8(12H) 3.3 - 3.9(4H) 3.67(3H,S) 3.97(2H.S) 5.13(1H,S) 6.73(1H,S) 7.1 - 8.2(4H) | HCl | 160 - 163 | -80.19 |
| 34 | 1.33(10H,S) 1.8 - 2.7(19H) 3.68(3H,S) 3.97(2H,S) 5.17(1H,S) 6.17(1H,S) 7.1 - 8.3(4H) | 2HCl | 159 - 163 | -72.89 |
| 35 | 1.33(10H,S) 2.13(2H,S) 2.1 - 2.6(14H) 3.10(2H,S) 3.67(3H,S) 3.97(2H,S) 5.15(1H,S) 6.07(1H,S) 7.1 - 8.2(9H) | 2HCl<br>3/1 acetone | 147 - 151 | -59.49 |
| 36 | 1.37(10H,S) 2.03(3H,S) 2.1 - 2.7(8H) 2.35(2H,S) 3.68(3H,S) 4.07(2H,S) 5.15(1H,S) 6.23(1H,S) 6.7 - 8.3(8H) | HCl | 142 - 144 | -77.49 |
| 37 | 0 83(3H,t) 1.33(10H,S) 2.27(3H,S) 2.35(3H,S) 2.37(2H,q) 3.47(2H,S) 3.63(3H,S) 4.03(2H,S) 5.12(1H,S) 6.22(1H,S) 7.0 - 8.2(9H) | HCl | 131 - 134 | -81.79 |
| | | | | |

Example 41

The compound (100 g) prepared in Example 1, lactose (421.25 g) and potato starch (50 g) were well mixed.

The mixture was poured into a fluidized bed granulator. Hydroxypropylcellulose (18.75 g) as a binding material was dissolved in water to make 5 % hydroxypropylcellulose solution which was then sprayed to the mixture to afford a granule.

Subsequently, potassium carboxymethylcellulose (20 g) serving as a disintegrator and magnesium stearate (15 g) serving as a lubricant were added to the resultant granule and mixed.

The obtained mixture was subjected to pressure molding to give a tablet (125 mg per one tablet).

Example 42

A tablet was prepared by the same manner as in Example 41 by employing the compound (100 g) obtained in Example 18.

[Pharmacological Test]

(1) Antihypertensive Action

Blood pressure was measured by oral administering the compound (10 mg/kg) obtained in each Example to each of two spontaneously hypertensive rats (weight: 250 - 350 g) in accordance with tail-cuff method (Hirofumi Sogabe). Measurement was conducted an hour after the administration. The difference between the blood pressure before the administration and that after the administration was observed for evaluating antihypertensive action. The results are shown in Table 3. The antihypertensive action of each of nidefipine and nicardipine was also measured for comparison.

Table 3

| Compound | Antihypertensive Action (mmHg) |
|---|---|
| Ex. No. 1 | -131 |
| 2 | - 89 |
| 3 | -109 |
| 5 | - 68 |
| 6 | -110 |
| 7 | - 61 |
| 8 | -140 |
| 9 | - 86 |
| 10 | -110 |
| 11 | - 74 |
| 12 | -113 |
| 13 | -108 |
| 15 | -114 |
| 16 | -120 |
| 17 | -112 |
| 18 | - 96 |
| 19 | - 75 |
| 20 | -129 |
| 21 | - 53 |
| 22 | -122 |
| 23 | - 87 |
| 24 | - 79 |
| 25 | - 98 |
| 26 | -111 |
| 27 | -109 |
| Comparison | |
| Nifedipine | - 58 |
| Nicardipine | - 77 |

(2) Calcium Antagonism

A thoracic aorta was removed from a rabbit to obtain a helical strips (width: 3 mm; length: 30 mm). This strip was suspended under a load of 0.5 g in a Magnus tube filled with Krebs-Henseleit solution. Subsequently, the Krebs-Henseleit solution was replaced with hyperpotassium solution (50 mM) to contract the strip. The strip was then left to stand until the contraction was stabilized. Thereafter, the compounds obtained in Examples 18, 19 and 20 and dissolved in ethanol-distilled water were added to the strip.

The concentration ($IC_{50}$) of each compound required for obtaining relaxation of 50 % was measured. It is to be noted that the relaxation of a strip treated with $10^{-4}$M papaverine was defined to 100 %. The results are shown in Table 4.

Table 4

| Compound (Ex. No.) | Measured Value ($IC_{50}$) |
|---|---|
| 18 | $100 \times 10^{-9}M$ |
| 19 | $1.5 \times 10^{-9}M$ |
| 20 | $420 \times 10^{-9}M$ |
| Nifedipine | $29 \times 10^{-9}M$ |
| Nicardipine | $6.8 \times 10^{-9}M$ |
| Manidipine | $27 \times 10^{-9}M$ |

(3) Acute Toxicity

After a fast, a ddy-mouse (4-week old) was orally given a solution obtained by dissolving and/or suspending the compound in each Example 18, 19 and 20 in dimethylsulfoxide (DMSO) or carboxymethylcellulose (CMC). The value of $LD_{50}$ was calculated from the mortality. The results are shown in Table 5.

Table 5

| Compound (Ex. No.) | Toxicity ($LD_{50}$) | Medium |
|---|---|---|
| 18 | 130 mg/kg | DMSO |
| 19 | 100 mg/kg | DMSO |
| 20 | 290 mg/kg | CMC |
| Nifedipine | 100 mg/kg | DMSO |
| Nicardipine | 190 mg/kg | DMSO |
| Manidipine | 200 mg/kg | CMC |

(4) Antihypertensive Action (regarding administration amount)

Ten spontaneously hypertensive rats (10 - 20 week old) were used for this test. Blood pressure was measured in accordance with tail-cuff method by employing a blood pressure gauge for small animals UR-1000 (manufactured by Ueda Works, Ltd., Japan). The compound of each Example 18, 19 and 20 was suspended in 0.5 % carboxymethylcellulose solution and orally administered to each rat. The amount of each compound required for lowering the blood pressure of 40 mmHg decrease in blood pressure was calculated from the relationship between the administration amount and the blood pressure after adiministration (lowermost blood pressure). This amount was defined as $ED_{-40}$ mmHg for serving as an index of antihypertensive action. $T_{\frac{1}{2}}$ was shown as the time required for recovery to 50 % of maximal hypertension. This value was used for an index of persistence. The results are shown in Table 6.

Table 6

| Compound (Ex. No.) | $ED_{-40}$ mmHg | $T_{1/2}$(dose) |
|---|---|---|
| 18 | 0.7 mg/kg | >6.0 hr(3 mg/kg)* |
| 19 | 0.5 mg/kg | >6.0 hr(3 mg/kg)* |
| 20 | 0.3 mg/kg | >6.0 hr(1 mg/kg)* |
| Nifedipine | 3.3 mg/kg | 4.0 hr(10mg/kg)* |
| Nicardipine | 2.6 mg/kg | 4.5 hr(10mg/kg)* |
| Manidipine | 1.7 mg/kg | 5.0 hr(3 mg/kg)* |

## Claims

1. A compound represented by the formula (I):

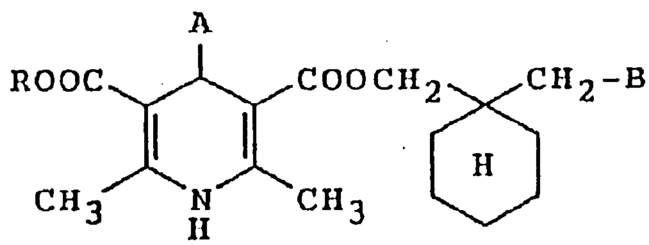

(I)

where A is a phenyl group substituted at least with a nitro group; B is a group represented by the formula:

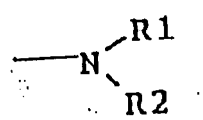

[where $R_1$ and $R_2$ are, the same or different, a lower alkyl group or a benzyl group which may be substituted with a halogen atom, or they may be combined, together with the adjacent nitrogen atom, through or not through an oxygen atom to form a six-membered ring or through a nitrogen atom to form a group represented by the formula:

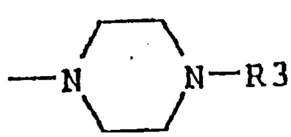

(where $R_3$ is a lower alkyl group, a phenyl or benzyl group which may be substituted with a halogen atom, or a diphenylmethyl group)]; and R is a hydrogen atom or a lower alkyl group
, or a salt thereof.

2. A compound of claim 1, wherein A is m-nitrophenyl group, R is a lower alkyl group having 1 to 6 carbon atoms, and B is a group of the formula:

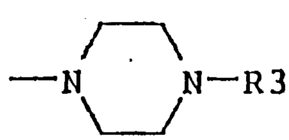

where $R_3$ is a lower alkyl group having 1 to 6 carbon atoms, or phenyl group, benzyl group or benzhydryl group, or a salt thereof.

**3.** A compound of claim 1, wherein A is m-nitrophenyl group, R is a lower alkyl group having 1 to 6 carbon atoms, and B is a group of the formula:

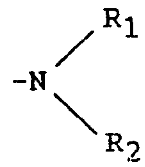

where $R_1$ and $R^2$ are the same or different a lower alkyl group having 1 to 6 carbon atoms or a benzyl group which may be substituted with a halogen atom, or a salt thereof.

**4.** A compound of claim 1 which is (+)-form or a salt thereof.

**5.** A compound of claim 1 which is (+)-1,4-dihydro-2,6-dimethyl-4-(3′-nitrophenyl)-3,5-pyridinedicarboxylic acid methyl 1-(4-phenylpiperadinylmethyl)cyclohexylmethyl ester or its racemate, or hydrochloride thereof.

**6.** A compound of claim 1 which is (+)-1,4-dihydro-2,6-dimethyl-4-(3′-nitrophenyl)-3,5-pyridinedicarboxylic acid 1-(N-benzyl-N-methylaminomethyl)cyclohexylmethyl methyl ester or its racemate, or hydrochloride thereof.

**7.** A compound of claim 1 which is (+)-1,4-dihydro-2,6-dimethyl-4-(3′-nitrophenyl)-3,5-pyridinedicarboxylic acid methyl ester 1-(piperidinylmethyl)cyclohexylmethyl ester or its racemate, or hydrochloride thereof.

**8.** A pharmaceutical composition which comprises a compound of the formula (I) as defined in claim 1 or its pharmaceutically acceptable salt and a pharmaceutically acceptable excipient or carrier.

**9.** A composition of claim 8 in which the compound of the formula (I) is any one as claimed in any of claims 2 to 7.

**10.** A method for preparing a compound of the formula (I) as defined in claim 1 or a salt thereof, which comprises reacting a compound of the formula (II):

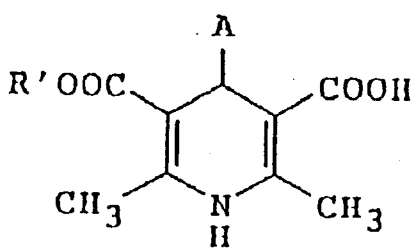

(II)

where A is the same meaning as in the formula (I); and R′ represents a lower alkyl group or a protecting group for carboxyl group
,or a reactive derivative thereof with a compound of the formula (III):

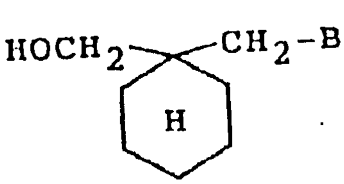

(III)

where B is the same meaning as in the formula (I) ,or a reactive derivative thereof, and then removing the protecting group for a carboxyl group if necessary.

**11.** A process for preparing a compound of the formula (I) as defined in claim 1 or a salt thereof, which comprises simultaneously reacting the following three compounds: a compound of the formula (IV):

A-CHO (IV)

where A is the same meaning as in the formula (I) ,a compound of the formula (V):

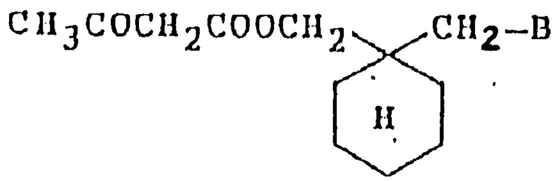

(V)

where B is the same meaning as in the formula (I) ,and β-aminocrotonic acid alkyl ester, or reacting first the compound of the formula (IV) with the compound of the formula (V) to afford a compound of the formula (VI):

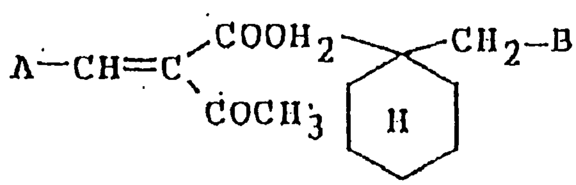

(VI)

where A and B are the same meanings as in the formula (I) ,and reacting then β-aminocrotonic acid alkyl ester with the compound of the formula (VI).

**12.** A process for preparing a compound of the formula (I) as defined in claim 1 or a salt thereof, which comprises reacting a compound of the formula (VII):

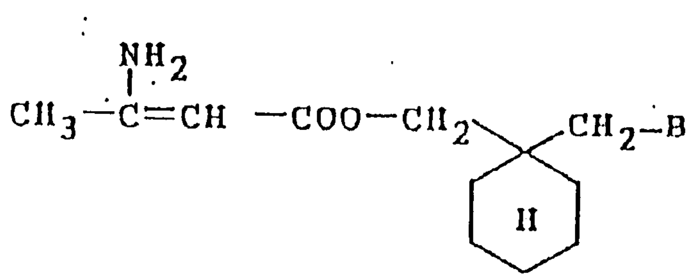

(VII)

where B is the same meaning as in the formula (I) ,with a compound of the formula (VIII):

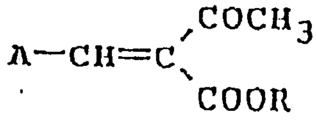

(VIII)

where A and R are the same meanings as in the formula (I).